(19) Europäisches Patentamt
European Patent Office
Office européen des brevets

(11) Publication number: **0 348 124 B1**

(12) # EUROPEAN PATENT SPECIFICATION

(45) Date of publication of patent specification: **27.04.94**

(51) Int. Cl.5: **C07D 471/04**, //(C07D471/04, 221:00,205:00)

(21) Application number: **89306173.9**

(22) Date of filing: **19.06.89**

(54) **Intermediate for 1-carba(dethia)cephalosporin.**

(30) Priority: **22.06.88 US 209596**

(43) Date of publication of application:
**27.12.89 Bulletin 89/52**

(45) Publication of the grant of the patent:
**27.04.94 Bulletin 94/17**

(84) Designated Contracting States:
**AT BE CH DE ES FR GB GR IT LI NL SE**

(56) References cited:
**EP-A- 0 175 544**
**WO-A-87/01116**
**US-A- 4 208 515**
**US-A- 4 782 144**

(73) Proprietor: **ELI LILLY AND COMPANY**
**Lilly Corporate Center**
**Indianapolis Indiana 46285(US)**

(72) Inventor: **Bodurow, Chistina Clara**
**5671 Crittenden**
**Indianapolis Indiana 46220(US)**

(74) Representative: **Tapping, Kenneth George et al**
**Erl Wood Manor**
**Windlesham Surrey, GU20 6PH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

This invention relates to β-lactam antibiotics. In particular, it relates to p-nitrobenzyl 7β-amino-3-hydroxy-1-carba(dethia)-3-cephem-4-carboxylate which is useful in the preparation of 1-carba(dethia)-cephalosporin antibiotics.

The 1-carba(dethia)cephalosporins have been obtained by total synthesis in contrast to the cephalosporin antibiotics which, with few exceptions, are produced by semi-synthetic methods. Evans et al., U.S. Patent No. 4,665,171, describe an asymmetric synthesis of 1-carba(dethia)cephalosporin intermediates. S. Uyeo and H. Ona, Chem. Pharm. Bull. (Japan), 28, 1563-1577 (1980), disclose a synthesis of certain racemic 1-carba(dethia)cephalosporinsand Hirata et al., U.S. Patent No. 4,708,956, describe 3-halo-1-carba-(dethia)cephalosporins and a method for the preparation thereof.

US4208515 discloses 7-Phenoxy and 7-phenylthio-acetamido-3-halo-3-cephem derivs. useful as broad spectrum antibacterials, EP-A-175544 describes the preparation of 7-amino-3-(1-pyridinium-methyl)-3-cephem-4-carboxylate as di-hydrochloride di-hydrate, WO8701116 discloses stable crystalline cephalosporin intermediates, prepared by de-silylating with alkanol then acidifying and US4782144 discloses crystalline 1-carba(dethia)-3-hydroxy 3-cephem esters, useful as intermediates for antibiotics.

The 1-carbacephalosporins are increasing in importance as new members of the β-lactam class of antibiotics for use in the treatment of infectious diseases. Since these antibiotics are obtained by total synthesis, purified intermediates useful in the preparation thereof are much sought after.

p-Nitrobenzyl 7-amino-3-hydroxy-1-carba(dethia)-3-cephem-4-carboxylate is provided in the form of the hydrochloride salt. The intermediate has been obtained in purified form by the N-deacylation of p-nitrobenzyl 7-phenoxyacetylamino-3-hydroxy-1-carba(dethia)-3-cephem-4-carboxylate. The crystalline salt is useful as an intermediate for preparing 1-carbacephalosporin antibiotics.

This invention provides p-nitrobenzyl 7-amino-3-hydroxy-1-carba(dethia)-3-cephem-4-carboxylate hydrochloride represented by formula 1.

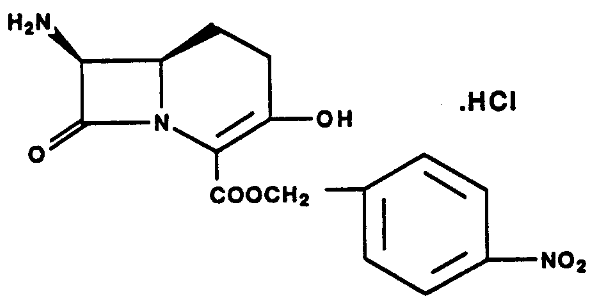

.HCl

1

The salt 1 is provided as a solid in a high state of purity.

The 3-hydroxy-1-carbacephalosporin ester 1 has thus far been obtained only by the N-deacylation of a 7-acylamino-3-hydroxy-1-carba(dethia)-3-cephem-4-carboxylic acid p-nitrobenzyl ester. For example, during the attempted two-step chlorination and N-deacylation of p-nitrobenzyl 7-phenoxyacetylamino-3-hydroxy-1-carba(dethia)-3-cephem-4-carboxylate by the method described by Hatfield et al., U.S. Patent No. 4,226,986, N-deacylation occurred without concomittant chlorination of the 3-hydroxy group to form 1. The N-deacylation was carried out with 2 moles of triphenylphosphite-chlorine adduct prepared at -30 °C in dichloromethane. The chlorination reagent was mixed with a solution of 3-hydroxy 1-carba ester in the presence of pyridine and under nitrogen. The mixture was stirred for 4 hours at -30 °C to form the imino chloride, isobutanol was added and the temperature of the mixture rose to -14 °C. Hydrogen chloride was passed into the mixture which was then warned to 25 °C. The 3-hydroxy nucleus ester hydrochloride salt precipitated and was slurried in 3A ethanol to remove any residual pyridinium hydrochloride. The salt 1 was obtained as a white solid on filtering the slurry.

The N-deacylation of a 7-acylamino-3-hydroxy-1-carba(dethia)-3-cephem-4-carboxylic acid p-nitrobenzyl ester, e.g., 7-phenylacetylamino or 7-phenoxyacetylamino 3-hydroxy ester, can be carried out with other imino chloride-forming reagents to form 1 without chlorination of the 3-hydroxy group. Imino chloride reagents such as $PCl_5$, $PCl_3$, $POCl_3$, $SOCl_2$ and the like, can be used in the deacylation. The 3-hydroxy group of the 1-carba-3-hydroxycephalosporins appears to be less reactive toward chlorination with these reagents than the 3-hydroxy group of the 3-hydroxycephalosporins. Accordingly, the N-deacylation method allows for selective formation of the 7-amino-3-hydroxy-1-carba-3-cephem p-nitrobenzyl ester.

The p-nitrobenzyl 7-acylamino-3-hydroxy-1-carba(dethia)-3-cephem-4-carboxylate, e.g., the 7-phenoxyacetylamino ester is prepared by the method of Evans et al., U.S. Patent No. 4,665,171.

The 7-amino-3-hydroxy ester hydrochloride salt 1 is somewhat hygroscopic; however, it can be readily protected from conditions of high humidity by storage in a tightly closed container or in a dry atmosphere.

The salt 1 can be converted to the free 7-amino ester by neutralization with base and the 7-amino ester reacylated with the desired carboxylic acid. For example, the free 7-amino ester can be acylated with D-phenylglycyl chloride hydrochloride to form p-nitrobenzyl 7$\beta$-(D-phenylglycylamino)-3-hydroxy-1-carba-(dethia)-3-cephem-4-carboxylate. The latter then may be converted via the 3-trifluoromethylsulfonyloxy derivative (triflate) to the 3-chloro ester as taught by Evans et al., U.S. Patent No. 4,673,737.

Alternatively, the amino group of the free 7-amino-3-hydroxy ester obtained from salt 1 can be protected with a conventional amino-protecting group such as, for example, the t-BOC group or the benzyloxycarbonyl (CBz) group. The 7-protected-amino-3-hydroxy ester can be converted to the 3-chloro derivative via the 3-triflate ester as described by Evans supra. After chlorination the amino-protecting group is removed and the unprotected 7-amino group is acylated with the carboxylic acid providing the acyl group of the desired final product, e.g., phenylglycine.

Following either of the above alternative synthesis, the p-nitrobenzyl ester group is removed, e.g., by zinc and HCl reduction or by catalytic hydrogenolysis over 5% palladium-on-carbon catalyst. The deesterified free acid is the desired antibiotic.

The following Example is provided to further illustrate and describe the invention.

Example 1

Preparation of p-nitrobenzyl 7$\beta$-amino-3-hydroxy-1-carba(dethia)-3-cephem-4-carboxylate hydrochloride

To an oven-dried 250-ml three-necked flask equipped with a stir bar, rubber septum, thermometer and nitrogen inlet was added dichloromethane (100 ml) and pyridine (1.73 ml). The flask was cooled to -30 °C and chlorine gas bubbled through the solution. The solution turned light yellow and triphenylphosphite (14.48 g, 46.7 mmole, 2.18 eq.) was added via syringe at such a rate as to maintain the yellow color and keep the internal temperature below -15 °C. An additional 20 drops of triphenylphosphite was added at the end of addition to disperse the yellow color. The solution was stirred at -30 °C for 20 minutes.

To an oven-dried 250-ml three-necked round bottom flask equipped with thermometer, stir bar, nitrogen inlet and rubber septum was added p-nitrobenzyl 7$\beta$-phenoxyacetylamino-3-hydroxy-1-carba(dethia)-3-cephem-4-carboxylate (10 g, 21.4 mmole), ethyl acetate (100 ml) and pyridine (2.1 ml). The solution was cooled to -25 °C and the triphenylphosphite dichloride solution from above added all at once via cannula under nitrogen. The reaction was stirred for 4 hours at -30 °C. At that time, isobutanol (20 ml) was added all at once and the internal temperature rose from -30 °C to -14 °C. Hydrogen chloride was bubbled through for 40 seconds and the ice bath was removed. The reaction was warmed to 25 °C with stirring. The solid was collected, washed with a cold solution of 1:1 ethyl acetate and dichloromethane and dried in vacuo for 48 hours to give 5.60 g of a white solid. The solid was slurried in 90 ml of 3A ethanol for 1 hour to remove any residual pyridinium hydrochloride. The resulting suspension was filtered, the purified solid dried in vacuo to give the desired product: 4.91 g (58%).

Decomposition pt: 170-190 °C.

$^1$HNMR ($d_6$-DMSO): $\delta$ 2.06 (m, 2H, $-CH_2-$), 2.60 (m, 2H, $-CH_2-$), 3.95 (m, 1H, N-C-CH), 4.8 (d, 1H, $H_2N$-CH), 5.4 (q, 2H, $CO_2CH_2-$), 8.0 (q, 4H, aromatic CH), 9.4 (br s, 2H, $H_2N$).

IR (KBr disk): 3450 (br, NH), 2596 (s, OH), 1770 (s, CO), 1755 (s, CO), 1654 (-C(O)-CH = CH-OH) $cm^{-1}$.

UV (EtOH) $\lambda_{max}$ 276 (absorbance 2,377)

Mass Spectrum (Field Desorption): m/e 333

$$[\alpha]^{25}_{589} + 11.99$$

| Analysis calculated for: $C_{15}H_{16}N_3O_6Cl$: | | | | |
|---|---|---|---|---|
| Theory | C, 48.73; | H, 4.36; | N, 11.36; | Cl, 9.59 |
| Found | C, 48.70; | H, 4.23, | N, 11.14; | Cl, 9.66 |

## Claims

### Claims for the following Contracting States : AT, BE, CH, DE, FR, GB, GR, IT, LI, NL, SE

1. The compound of the formula

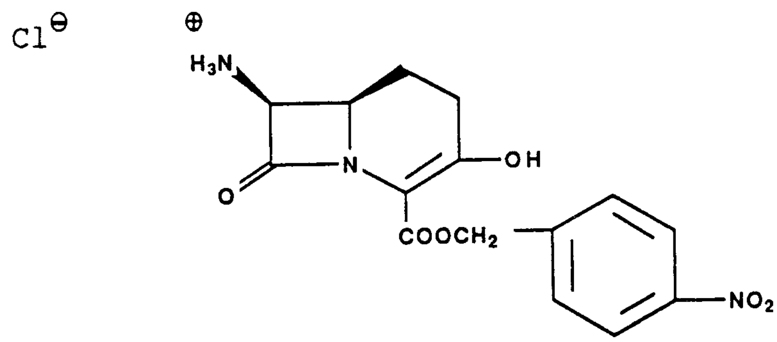

in substantially pure form.

2. The process for preparing the compound according to claim 1 which comprises reacting a compound of the formula

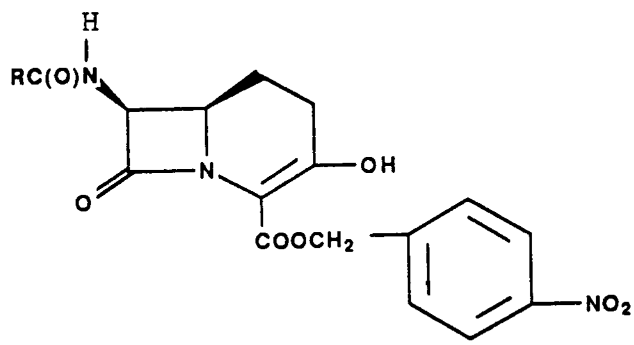

wherein R is benzyl or phenoxymethyl with an imino chloride-forming reagent and, thereafter, with an imino ether-forming alcohol.

3. The process according to claim 2 wherein the imino chloride-forming reagent is a phosphorus chloride.

4. The process according to claim 3 wherein the phosphorus chloride is phosphorus pentachloride, phosphorus trichloride or phosphorus oxychloride.

5. The process according to claim 2 wherein the imino chloride-forming reagent is triphenylphosphite-chlorine adduct.

6. The process according to claim 5 when the imino chloride-forming step is carried out at a temperature of about -30 °C.

**Claims for the following Contracting State : ES**

1. The process for preparing the compound of the formula

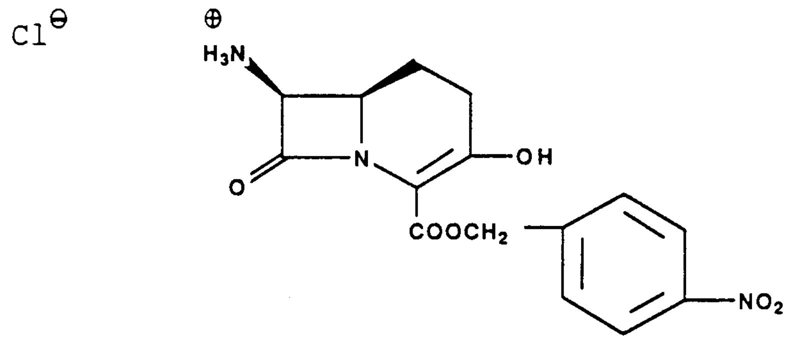

which comprises reacting a compound of the formula

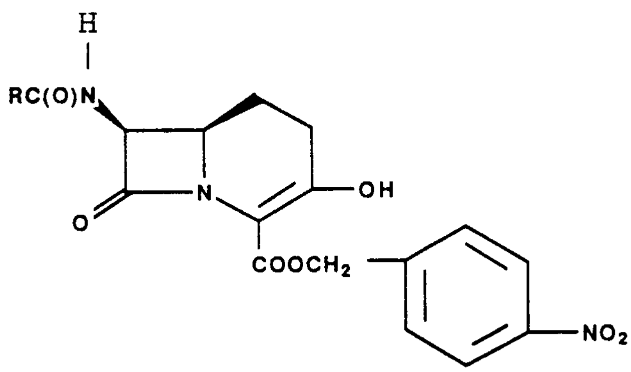

wherein R is benzyl or phenoxymethyl with an imino chloride-forming reagent and, thereafter, with an imino ether-forming alcohol.

2. The process according to claim 1 wherein the imino chloride-forming reagent is a phosphorus chloride.

3. The process according to claim 2 wherein the phosphorus chloride is phosphorus pentachloride, phosphorus trichloride or phosphorus oxychloride.

4. The process according to claim 1 wherein the imino chloride-forming reagent is triphenylphosphite-chlorine adduct.

5. The process according to claim 4 when the imino chloride-forming step is carried out at a temperature of about -30 ° C.

## Patentansprüche

### Patentansprüche für folgende Vertragsstaaten : AT, BE, CH, DE, FR, GB, GR, IT, LI, NL, SE

1. Verbindung der Formel

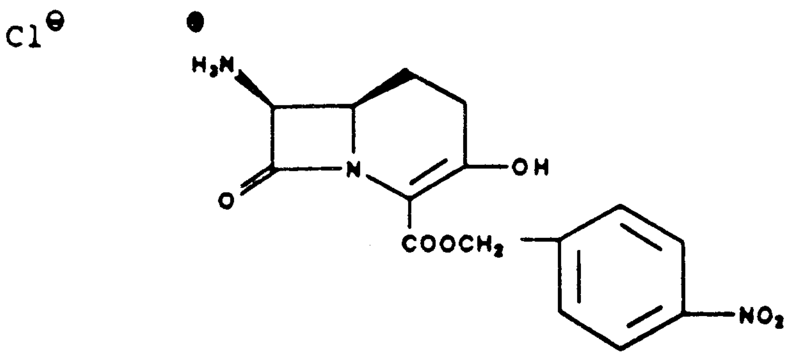

in im wesentlichen reines Form.

2. Verfahren zur Herstellung der Verbindung nach Anspruch 1 umfassend, daß man eine Verbindung der Formel

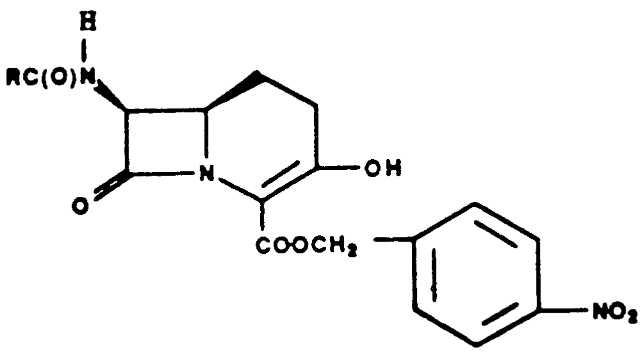

worin R ein Benzyl oder Phenoxymethylrest ist, mit einem Iminochlorid bildenden Reagenz und danach mit einem Iminoether bildenden Alkohol umsetzt.

3. Verfahren nach Anspruch 2, worin das Iminochlorid bildende Reagenz ein Phosphorchlorid ist.

4. Verfahren nach Anspruch 3, worin das Phosphorchlorid Phosphorpentachlorid, Phosphortrichlorid oder Phosphoroxychlorid ist.

5. Verfahren nach Anspruch 2, worin das Iminochlorid bildende Reagenz ein Triphenylphosphit-Chloraddukt ist.

6. Verfahren nach Anspruch 5, wobei die Iminochlorid bildende Stufe bei einer Temperatur von etwa -30 ° C durchgeführt wird.

**Patentansprüche für folgenden Vertragsstaat : ES**

1. Verfahren zur Herstellung einer Verbindung der Formel

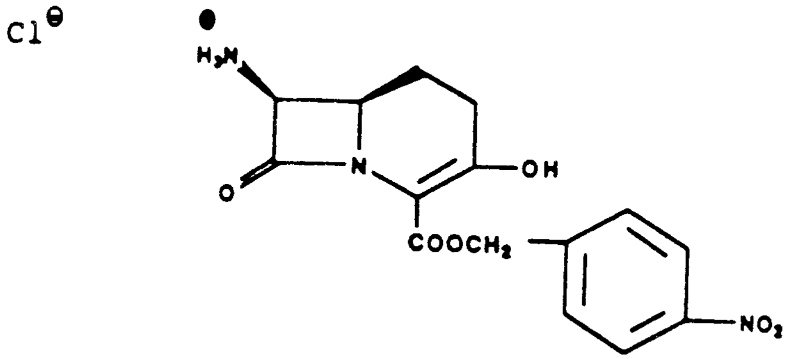

umfassend, daß man eine Verbindung der Formel

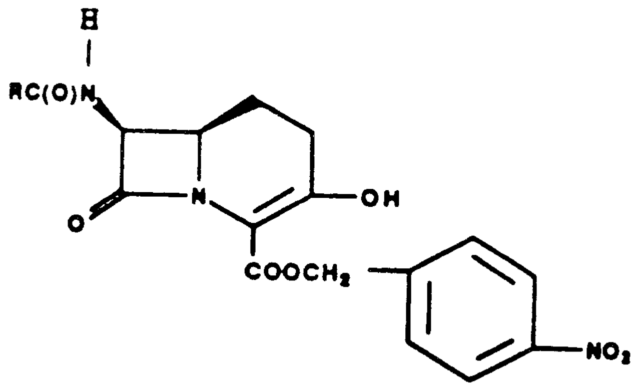

worin R ein Benzyl- oder Phenoxymethylrest ist, mit einem Iminochlorid bildenden Reagenz und danach mit einem Iminoether bildenden Alkohol umsetzt.

2. Verfahren nach Anspruch 1, worin das Iminochlorid bildende Reagenz ein Phosphorchlorid ist

3. Verfahren nach Anspruch 2, worin das Phosphorchlorid Phosphorpentachlorid, Phosphortrichlorid oder Phosphoroxychlorid ist.

4. Verfahren nach Anspruch 1, worin das Iminochlorid bildende Reagenz ein Triphenylphosphit-Chloraddukt ist.

5. Verfahren nach Anspruch 4, wobei die Iminochlorid bildende Stufe bei einer Temperatur von etwa -30 ° C durchgeführt wird.

**Revendications**

**Revendications pour les Etats contractants suivants : AT, BE, CH, DE, FR, GB, GR, IT, LI, NL, SE**

**1.** Composé de formule

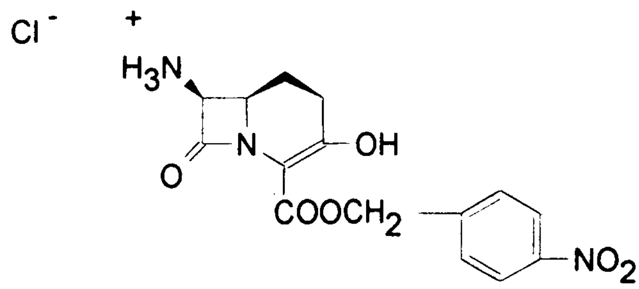

sous forme substantiellement pure.

**2.** Procédé pour la préparation du composé selon la revendication 1 comprenant la réaction d'un composé de formule

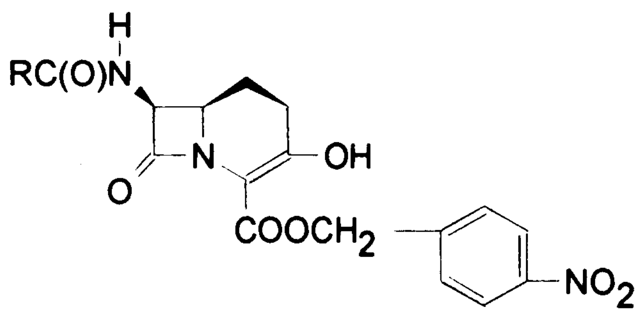

dans laquelle R est un benzyle ou un phénoxyméthyle, avec un réactif formant un iminochlorure, et ensuite avec un alcool formant un iminoéther.

**3.** Procédé selon la revendication 2, où le réactif formant l'iminochlorure est un chlorure du phosphore.

**4.** Procédé selon la revendication 3, où le chlorure de phosphore est le pentachlorure de phosphore, le trichlorure de phosphore ou l'oxychlorure de phosphore.

**5.** Procédé selon la revendication 2, où le réactif formant l'iminochlorure est le précurseur du triphénylphosphite-chlore.

**6.** Procédé selon la revendication 5, lorsque l'étape de formation de l'iminochlorure est effectuée à une température d'environ -30 ° C.

**Revendications pour l'Etat contractant suivant : ES**

1. Procédé pour la préparation du composé de formule

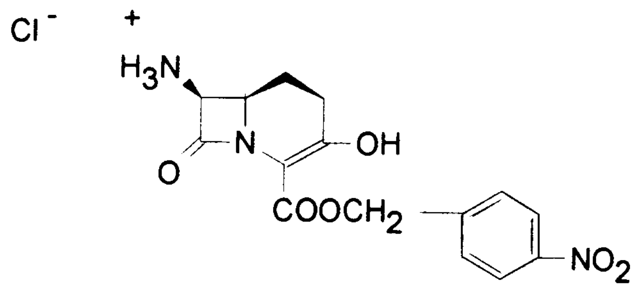

comprenant la réaction d'un composé de formule

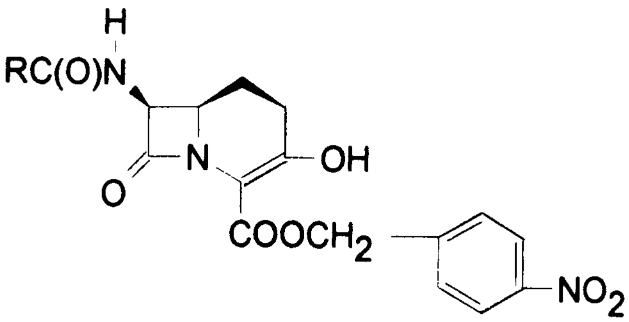

dans laquelle R est un benzyle ou un phénoxyméthyle, avec un réactif formant un iminochlorure, et ensuite avec un alcool formant un iminoéther.

2. Procédé selon la revendication 1, où le réactif formant l'iminochlorure est un chlorure du phosphore.

3. Procédé selon la revendication 2, où le chlorure de phosphore est le pentachlorure de phosphore, le trichlorure de phosphore ou l'oxychlorure de phosphore.

4. Procédé selon la revendication 2, où le réactif formant l'iminochlorure est le précurseur du triphénylphosphite-chlore.

5. Procédé selon la revendication 4, lorsque l'étape de formation de l'iminochlorure est effectuée à une température d'environ -30 ° C.